# EUROPEAN PATENT SPECIFICATION

(11) **EP 1 829 579 B1**
(45) Date of publication and mention of the grant of the patent: **21.01.2009**
(21) Application number: 07002925.1
(22) Date of filing: 12.02.2007
(51) Int. Cl.: A61M 39/02, A61M 39/22, A61M 39/04, A61M 5/14

(54) **Liquid co-infusion device**
Flüssigkeit-Koinfusionsgerät
Dispositif d'infusion liquide

(30) Priority: 03.03.2006 JP 2006058262
(43) Date of publication of application: 05.09.2007
(73) Proprietor: Covidien AG, 8212 Neuhausen am Rheinfall (CH)
(72) Inventor: Kitani, Ichiro, Fukuroi-shi, Shizuoka-ken (JP); Funamura, Shigeaki, Fukuroi-shi, Shizuoka-ken (JP)
(74) Representative: Tomlinson, Edward James

(56) References cited:
- EP-A1- 0 576 380
- WO-A-00/40291
- WO-A-20/05021084
- BE-A- 386 964
- FR-A1- 2 804 609

## Description

### Technical field

The present invention relates to a liquid co-infusion device for medical treatment which has multiple branch tubes connected to multiple transfusion tubes or the like, and which switches between connecting and disconnecting the branch tubes.

In the past, a specific physiological saline, liquid medicine or the like would have been administered to a patient using multiple transfusion tubes. In such cases, a liquid co-infusion device would be used and the operation carried out by connecting or disconnecting the various transfusion tubes. Among such liquid co-infusion devices are those that have three branch tubes. The liquid co-infusion device is constituted with a chamber part that connects to the three branch tubes and a valve body that is rotated inside the chamber part by operating an operating part. By operating the operating part to rotate the valve body, any of the branch tubes can be connected or disconnected.

However, with the abovementioned liquid co-infusion device, the operating part provided projects from the circumferential surface of the chamber part, and the tube members connected to the branch tubes sometimes get tangled on the operating part, or the patient sometimes touches the operating part and turns it. For this reason, a liquid co-infusion device that can switch among branch tube channels has been developed such that the chamber part is cylindrical, with the operating part not projecting from the circumferential surface of the chamber part, by the operating part being movable in the axial direction of the chamber part to connect or disconnect a specific branch tube to or from the chamber part (for example, see Japanese Kokai Patent Application No. Sho 62[1987]-172962). This liquid co-infusion device is constituted such that two branch tubes are furnished, one on each side of the circumferential surface of a cylindrical chamber part, so that the two branch tubes can be opened and closed by rotating the valve body around its axis inside the chamber part.

However, this liquid co-infusion device has only two branch tubes and can only connect or disconnect two branch tubes. Therefore, with this liquid co-infusion device, it is not possible to connect multiple transfusion tubes or the like used for medical treatment, and to switch from connecting or disconnecting the various transfusion tubes. There is also the problem that the valve body must be rotated to connect or disconnect the two branch tubes, so that operating it is difficult.

A device with the features of the preamble of claim 1 is disclosed in document BE 386 964.

### Summary of the invention

The present invention was devised taking these circumstances into consideration. Its objective is to provide a liquid co-infusion device with which any of multiple branch tubes can be connected or disconnected without the operating part projecting and getting in the way.

In order to achieve the abovementioned objective, a feature in the constitution of the liquid co-infusion device pertaining to the present invention is that it has: a liquid co-infusion device body composed of a chamber part formed in the shape of a tube, a branch tube formed at one end in the axial direction of the chamber part and that has a chamber that can be connected to or disconnected from the interior of the chamber part, and a pair of branch tubes that extend outward from two sides of the outer circumferential surface of the chamber part and each of which has a channel that connects to or disconnects from the interior of the chamber part; and an operating part that is arranged at the other end in the axial direction of the chamber part to be movable in the axial direction of the chamber part, and which has a valve body that switches between connection or disconnection of one of the pair of branch tubes and the interior of the chamber part by being positioned toward the interior of the chamber part or toward the outside of the chamber part.

The liquid co-infusion device of the present invention that is constituted as described above has an operating part with a valve body that can move in the axial direction of the chamber part. By operating the operating part, one of the pair of branch tubes and the interior of the chamber part can be connected or disconnected. The branch tube formed at one end in the axial direction of the chamber part has a channel that can be connected to or disconnected from the interior of the chamber part. Therefore, by operating the operating part, the pair of branch tubes can be connected through the chamber part, and by connecting the channel of the branch tube at one end of the chamber part to the chamber part, the three branch tubes can all be connected through the chamber part.

Because of this, two types of medicine or the like can be administered to the patient. Also, by disconnecting the channel of a branch tube at one side of the chamber part from the chamber part in this state, only a pair of branch tubes can be connected. In addition, by connecting one of the pair of branch tubes and the interior of the chamber part with the channel in the branch tube at the end of the chamber part connected to the chamber part, the branch tube at the end of the chamber part and the other of a pair of branch tubes can be connected. In this case, the pair of branch tubes could also be connected to each other when the valve body is positioned toward the inside in the axial direction of the chamber part, and a pair of branch tubes can also be connected to each other when the valve body is positioned toward the outside in the axial direction of the chamber part.

Using this liquid co-infusion device, switching between connection and disconnection of the pair of branch tubes is accomplished just by moving the operating part in the axial direction of the chamber part. This is simple, and the position of the operating part is also easy to determine visually, so mishandling does not occur easily. Also, the operating part only advances or withdraws in the axial direction relative to the chamber part, so it does not project from the circumferential surface of the chamber part to get in the way. In addition, when this type of liquid co-infusion device is placed on a bed or the like, the axial direction of the chamber part is oriented horizontally, so the position in which the operating part can be switched by contact with the patient also does not occur.

Here, the shape of the chamber part in this case may be that of a round tube, a square tube, a triangular tube, an elliptical tube, a semicircular tube, or any other tube shape that extends in the axial direction. The valve body used may be formed by cutting away one side of a round columnar body such that it can close only one of the pair of branch tubes, or by forming a channel composed of a hole inside a round columnar body.

Another feature in the constitution of a liquid co-infusion device pertaining to the present invention is that a guiding part is furnished in the section of the chamber part where the operating part is disposed, a guided part is furnished for the operating part that can move relative to the guiding part when locked to the guiding part, and the operating part moves relative to the chamber part by moving the guided part relative to the guiding part.

Because of this, the operating part moves in a precise path formed by the relative movement with the guiding part and the guided part locked, so that channel switching is reliable. In this case, the guiding part and the guided part can be constituted with, for example, a slot and a locking projection that can move while sliding locked within the slot, and the guiding part and the guided part could be furnished in either the chamber part or the operating part. In addition, the guiding part and the guided part could also be furnished at any section of the chamber part of the operating part. In short, the chamber part and the operating part need only be able to move relatively via the guiding part and the guided part.

Still another feature in the constitution of a liquid co-infusion device pertaining to the present invention is that the operating part has a grip part that can move in the axial direction along the outer circumferential surface of the section on said other end, in the axial direction, of the chamber part. Because of this, the operator operating the liquid co-infusion device holds the grip part to move the operating part in the axial direction of the chamber part so that switching is performed using the valve body. Also, in this case, because the grip part can move along the outer circumferential surface of the chamber part, operation is easy.

Still another feature in the constitution of a liquid co-infusion device pertaining to the present invention is that the guiding part is furnished on the outer circumferential surface in a section at said other end, in the axial direction, of the chamber part, and the guided part is furnished in a section of the grip part corresponding to the guiding part. Because of this, formation of the guiding part and the guided part is easy. The operating part moves along a precise path by movement of the guided part along the guiding part, and channel switching is reliable.

Still another feature in the constitution of a liquid co-infusion device pertaining to the present invention is that the guided part is constituted with a slot or a through hole that has a section that extends in the axial direction of the chamber part, and the guiding part is constituted with a projection that locks into the slot or through hole to be able to move in it. Also, in this case, a narrow part for positioning the projection at the two ends of the slot or through hole is furnished near the two ends of the slot or the through hole that constitutes the guiding part. Because of this, the projection locks in the narrow part and is restrained, so that the channel is prevented from being changed by unintentionally moving the projection.

Still another feature in the constitution of a liquid co-infusion device pertaining to the present invention is that a window to expose the circumferential surface of the valve body is formed in a specific section of the chamber part, and marks that indicate the channel that will be connected are furnished on the circumferential surface of the valve body at the section exposed to the window when the valve body is positioned toward the inside in the axial direction of the chamber part and at the section exposed to the window when the valve body is positioned toward the outside in the axial direction of the chamber part. Because of this, the channel is indicated in the window, and the channels that are to be connected or disconnected are not operated incorrectly.

Still another feature in the constitution of a liquid co-infusion device pertaining to the present invention is that a rubber stopper is attached to the branch tube formed at one end in the axial direction of the chamber part, and by passing a tubular insertion member into the rubber stopper, the interior of the chamber part and a liquid container connected to the tubular insertion member are connected through the branch tube formed at one end in the axial direction of the chamber part. Because of this, the channel in the branch tube formed at one end of the chamber part can be connected to or disconnected from the interior of the chamber part in a simple fashion. The tubular insertion member in this case is the inserted part (dull needle) of an adaptor, the male part of a luer syringe, or the like. When the tip of the tubular insertion member is not sharp, a slit for passing the tubular insertion member through can also be provided in the rubber stopper.

### Brief description of the figures

Embodiments of the invention will now be described by way of example only with reference to the accompanying figures in which:
Figure 1 shows the operating part of a liquid co-infusion device pertaining to a first embodiment of the present invention when in the up position. (a) is a plan view, (b) a front view, and (c) a side view;
Figure 2 shows the operating part of the liquid co-infusion device when in the down position. (a) is a plan view, (b) a front view, and (c) a side view;
Figure 3 is a cross section at 3-3 in Figure 1(a);
Figure 4 is a cross section at 4-4 in Figure 1(a);
Figure 5 is a cross section at 5-5 in Figure 2(a);
Figure 6 is a cross section at 6-6 in Figure 2(a);
Figure 7 is a cross section at 7-7 in Figure 2(b);
Figure 8 is a cross section showing a liquid co-infusion device to which an adaptor is attached and in which the operating part is in the up position, viewed from the front;
Figure 9 is a cross section showing a liquid co-infusion device to which an adaptor is attached and in which the operating part is in the up position, viewed from the side;
Figure 10 is a cross section showing a liquid co-infusion device to which an adaptor is attached and in which the operating part is in the down position, viewed from the front;
Figure 11 is a cross section showing a liquid co-infusion device to which an adaptor is attached and in which the operating part is in the down position, viewed from the side;
Figure 12 shows the operating part of a liquid co-infusion device pertaining to a second embodiment of the present invention, when in the up position. (a) is a plan view, (b) a front view, and (c) a side view;
Figure 13 is a front view showing the operating part of the liquid co-infusion device shown in Figure 12, in the down position;
Figure 14 is a cross section at 14-14 in Figure 12 (b);
Figure 15 is a cross section at 15-15 in Figure 12 (a);
Figure 16 is a cross section at 16-16 in Figure 12 (b);
Figure 17 is a cross section at 17-17 in Figure 13; and
Figure 18 is a cross section showing the liquid co-infusion device in Figure 13 viewed from the front.

### Embodiments of the invention

### First embodiment

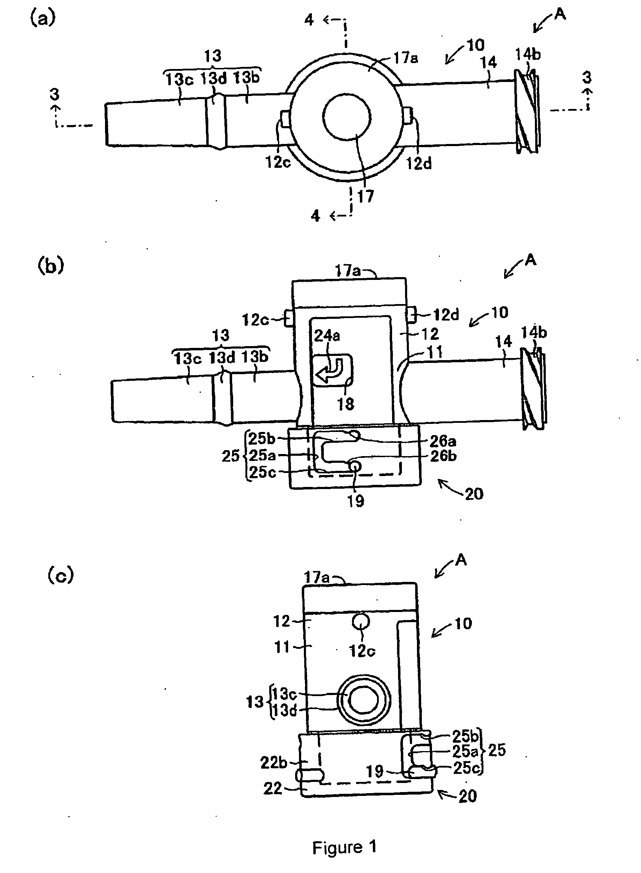

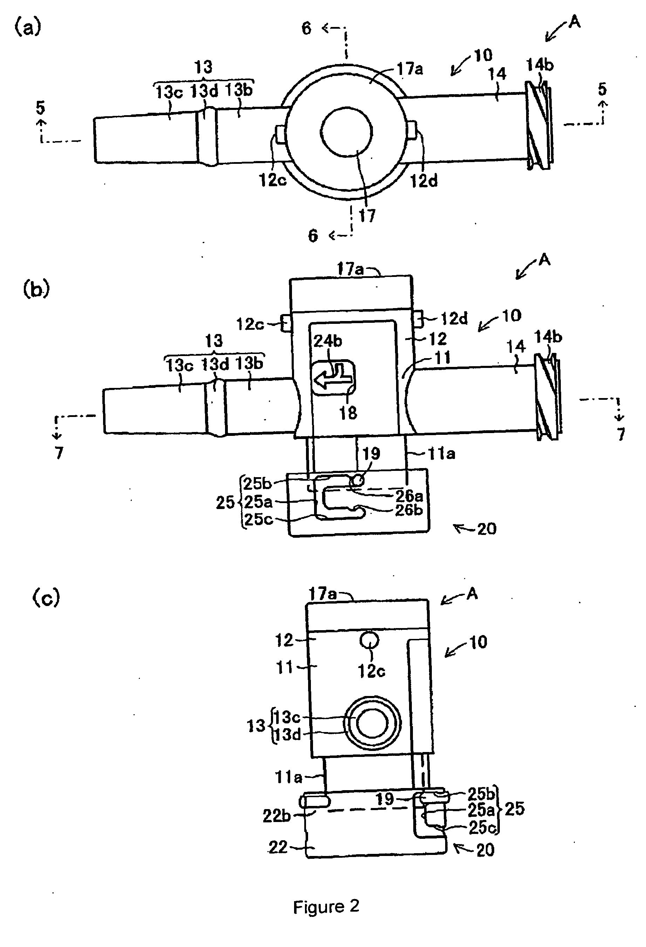

A liquid co-infusion device pertaining to the present invention will be explained in detail. Figures 1 and 2 show a liquid co-infusion device (A) pertaining to a first embodiment of the present invention. The liquid co-infusion device (A) is constituted with a liquid co-infusion device body (10) and an operating part (20). Liquid co-infusion device body (10) is constituted with a cylindrical chamber part (11) that is short in the axial direction, a flow merging branch tube (12) formed at one end (the top in Figures 1 and 2) in the axial direction of chamber part (11), and a downstream branch tube (13) and an upstream branch tube (14) that are connected on either side of the outer circumferential surface of chamber part (11) to extend along the same axis to maintain an angle of 180 degrees.

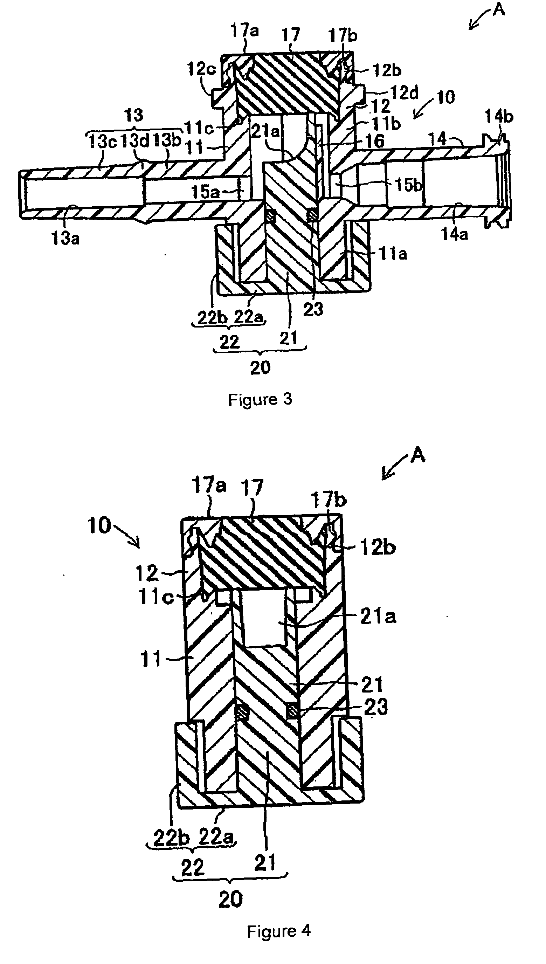

Chamber part (11) is formed as a stepped cylinder, such that the bottom section is narrower than the top part in the cross section viewed from the front surface as shown in Figure 3, and the thickness is uniform in the cross section viewed from the side surface as shown in Figure 4. Also, two through holes (15a) and (15b) are formed in the section facing the interface between small-diameter part (11 a) on the bottom and large-diameter part (11 b) on the top, this being positioned in the axial center of chamber part (11). Downstream branch tube (13) is furnished in the section of chamber part (11) corresponding to through hole (15a), and the inside of chamber part (11) and a channel (13a) formed inside downstream branch tube (13) are connected via through hole (15a).

Upstream branch tube (14) is formed in the section of chamber part (11) corresponding to through hole (15b), and the inside of chamber part (11) and channel (14a) formed inside upstream branch tube (14) are connected via through hole (15b). A vertical wall (16) to prevent backflow is also formed in the section of small-diameter part (11 a) of chamber part (11) corresponding to through hole (15b). Vertical wall (16) extends upward inside chamber part (11) from the top edge of the inner circumferential surface of small-diameter part (11 a), maintaining a separation from through hole (15b), so that medicine or the like flows through the top inside of chamber part (11) when medicine or the like flows from upstream branch tube (14) toward the inside of chamber part (11). Because of this, air or the like can be prevented from remaining in chamber part (11).

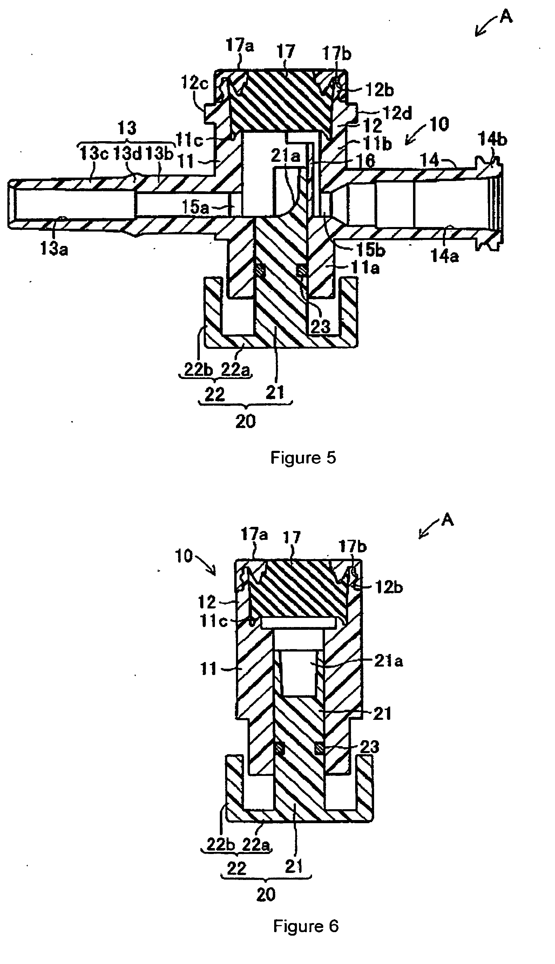

Therefore, with regard to vertical wall (16), when operating part (20) is as shown in Figures 5 and 6 (Figures 5 and 6 show valve body (21), described below, connecting chamber part (11) to upstream branch tube (14) by virtue of operating part (20) being in descended position), when medicine or the like is sent from upstream branch tube (14) toward the inside of chamber part (11), liquid rises along vertical wall (16) after passing through the inside of upstream branch tube (14) and through hole (15b) and spills over vertical wall (16) to flow into chamber part (11). In addition, the liquid flows to downstream branch tube (13) via through hole (15a).

Flow merging branch tube (12) formed at the top of chamber part (11) is formed in a cylindrical shape, the length of which in the axial direction is shorter than, and the diameter of which is larger than, downstream branch tube (13) or upstream branch tube (14), and it is constituted integrally with chamber part (11). A taper is furnished on the inner circumferential surface of flow merging branch tube (12) such that the diameter of the top opening is larger than the diameter toward chamber part (11). The internal diameter of flow merging branch tube (12) is larger than the internal diameter of chamber part (11), and a level difference is formed on the inside at the interface between the top end of chamber part (11) and the bottom end of flow merging branch tube (12). A shallow locking slot (11 c) is formed along the outer circumference of the level difference, and a rubber stopper (17) made of natural rubber or synthetic rubber is inserted inside flow merging branch tube (12) into locking slot (11c), thus closing off the inside of flow merging branch tube (12).

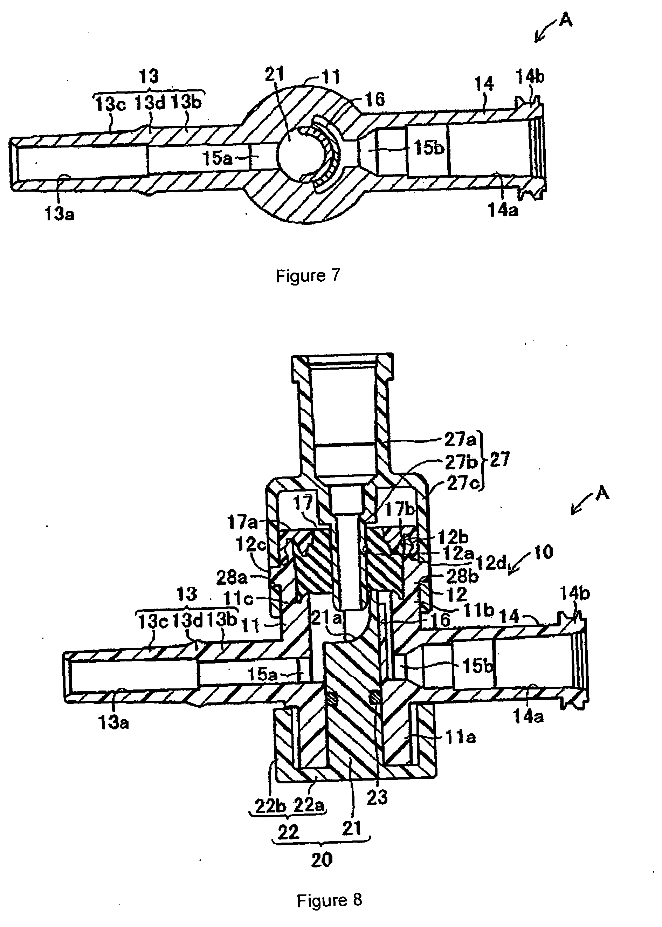

A slit (12a) (shown in Figures 8-11) that passes between the inside of chamber part (11) and the outside of flow merging branch tube (12) to form a channel in flow merging branch tube (12) is formed in rubber stopper (17). Slit (12a) is closed by the elasticity of rubber stopper (17) when flow merging branch tube (12) is not in use. A projecting part (12b), the outer circumferential surface of which is formed to be uneven, is also furnished on the top end of flow merging branch tube (12), and a cover (17a) that holds rubber stopper (17) in place in flow merging branch tube (12) is attached via projecting part (12b) in the spring part of flow merging branch tube (12).

Cover (17a) is formed approximately in a ring shape, the center of the top surface of which is open, and a locking recess (17b) that can lock onto projecting part (12b) is formed in the inner circumferential surface at the side. Therefore, cover (17a) presses the part of the top surface of rubber stopper (17) toward the outer circumference, and is attached in the opening of flow merging branch tube (12) by virtue of locking recess (17b) being locked onto projecting part (12b). A pair of locking projections (12c) and (12d) is also formed on the left and right side of the outer circumferential surface of flow merging branch tube (12) below projecting part (12b), and a small square window (18) is formed in the section of the circumferential surface of chamber part (11) somewhat toward downstream tube (13). In addition, a guiding projection (19) as the guiding part of the present invention is furnished below window (18) near the bottom end of the circumferential surface of chamber part (11).

Downstream branch tube (13) is formed integrally with chamber part (11) and is constituted with a base end (13b) positioned toward chamber part (11) and a male luer part (13c) positioned at the tip and formed to be narrower than base end (13b). Male luer part (13c) is formed with a pointed shape such that the tip is narrower than base end (13b). A projecting part (13d) is formed around on the outer circumferential surface of downstream tube (13) at the interface between base end (13b) and male luer part (13c).

Upstream branch tube (14) is formed integrally with chamber part (11), and a channel (14a) consisting of a tapered hole is formed inside. The channel (14a) is connected to through hole (15b), and the section toward through hole (15b) is tapered such that the diameter nearer through hole (15b) is smaller and the diameter farther from through hole (15b) is larger. The upstream section of channel (14a) is tapered such that the diameter gradually becomes larger closer to the opening in upstream branch tube (14). A threaded connecting part (14b) is formed on the outer circumference of the opening in upstream branch tube (14).

Operating part (20) is constituted with a valve body (21) that is approximately a round column, and a grip part (22) connected to the bottom end of valve body (21). Valve body (21) is disposed inside chamber part (11) and can move in the axial direction inside chamber part (11) by the operation of operating part (22). A slot for a seal member (23) is formed around the outer circumferential surface of valve body (21) in the section somewhat toward the bottom from the center. When valve body (21) moves inside chamber part (11), valve body (21) slides against the inner circumferential surface of small-diameter part (11 a) and vertical wall (16) and maintains a prescribed distance from the opening in channel (13a) of downstream branch tube (13). In this case, seal member (23) prevents leakage between valve body (21) and the inner circumferential surface of small-diameter part (11 a).

A curved surface (21a) that extends curving downward from flow merging branch tube (12) toward downstream branch tube (13) is formed in the section at the top of valve body (21) facing downstream branch tube (13). The curved surface (21 a), as shown in Figures 5 and 7, is formed such that when valve body (21) is at the bottom position, the surface bottom end is positioned at the bottom end of channel (13a) in downstream branch tube (13) and the top end is separated from rubber stopper (17). And as shown in Figure 3, curved surface (21 a) is formed such that when valve body (21) is at the top position, the surface bottom end is positioned somewhat above the top end of channel (13a) in downstream branch tube (13) and the top end presses against rubber stopper (17).

For this reason, as shown in Figure 3, when valve body (21) is at the top position, slit (12a) in flow merging branch tube (12) and channel (14a) in upstream branch tube (14) are both disconnected from chamber part (11). Although channel (13a) in downstream branch tube (13) is connected to chamber part (11), it is disconnected from slit (12a) in flow merging branch tube (12) and channel (14a) in upstream branch tube (14). Also, as shown in Figure 5, when valve body (21) is in the down position, channel (13a) in downstream branch tube (13) and channel (14a) in upstream branch tube (14) are both connected to chamber part (11) and connected to each other.

Therefore, in this state, medicine or the like can be sent from upstream branch tube (14) through chamber part (11) to downstream branch tube (13). A mark (24a) that indicates that the channel inside chamber (11) is turned from flow merging branch tube (12) toward downstream branch tube (13) is furnished on the section of the circumferential surface of valve body (21) at window (18) when valve body (21) is in the up position. Specifically, the mark (24a) indicates the state in which upstream branch tube (14) is disconnected from chamber part (11) and flow merging branch tube (12) can be connected to chamber part (11). A mark (24b) that indicates that the channel in chamber (11) is turned from upstream branch tube (14) toward downstream branch tube (13), and is turned from upstream branch tube (14) toward downstream branch tube (13) [sic] is furnished on the section of the circumferential surface of valve body (21) positioned at window (18) when valve body (21) is in the down position.

Grip part (22) is constituted with a disk-shaped bottom part (22a) that extends horizontally from the bottom end of valve body (21), and a cylindrical grip part body (22b) that extends upward along the outer circumferential surface of small-diameter part (11 a) of chamber part (11) from the outer circumferential edge of bottom part (22a). A guide slot (25) as the guided part of the present invention is formed in the section of grip part body (22b) corresponding to guiding projection (19). The guide slot (25) is formed with a width such that guiding projection (19) can move inside it along its length, and is constituted with a vertical guide (25a) that extends vertically, and horizontal guides (25b) and (25c) that extend horizontally parallel from the top end and bottom end, respectively, of vertical guide (25a).

Narrowed parts (26a) and (26b) are respectively formed at the ends of horizontal guides (25b) and (25c). The narrowed parts (26a) and (26b) are constituted with an elastic part such that the width of the narrowest part is somewhat smaller than the diameter of guiding projection (19). With a specific pressure, guiding projection (19) can be passed through this part by rotating operating part (20) so that guiding projection (19) moves from the center part to the end of horizontal guides (25b) and (25c). When guiding projection (19) is positioned at the end of horizontal guides (25b) and (25c), however, guiding projection (19) is securely held in that position by narrow parts (26a) and (26b).

Also, as shown in Figures 8-11, an adaptor (27) is detachably attached to flow merging branch tube (12). The adaptor (27) is constituted with a cylindrical female luer part (27a) in which a channel is formed on the inside, a narrow-diameter cylindrical insertion part (27b) as the luer member of the present invention that has a channel that connects with the channel in female luer part (27a), and a cover part (27c) that covers the perimeter of insertion part (27b). A male luer part connected to a transfusion tube (not shown) is inserted into female luer part (27a) and medicine or the like is sent through the transfusion tube. Female luer part (27a) and the inside of chamber part (11) are connected by inserting insertion part (27b) into slit (12a) in rubber stopper (17).

Cover part (27c) is constituted with a cylindrical body that extends downward to cover the circumferential surface of insertion part (27b), maintaining a constant spacing after widening horizontally from the bottom end of female luer part (27a). A pair of locking holes (28a) and (28b) that can lock onto projections (12c) and (12d) of flow merging branch tube (12) is formed in the facing sections near the bottom of the circumferential surface of cover part (27c). By locking the locking holes (28a) and (28b) onto projections (12c) and (12d) with insertion part (27b) inserted into slit (12a), insertion part (27b) can remain inserted into slit (12a). Also, in this case, insertion part (27b) and the circumferential surface of slit (12a) are pressed tightly together due to the elasticity of rubber stopper (17).

In this constitution, to supply a specific medicine to a patient (not shown), the back end of a transfusion tube (not shown), connected to an indwelling needle which pierces and remains in the patient, is connected to downstream branch tube (13). A male luer part, furnished at the tip of a transfusion tube that extends from a container or the like storing the medicine to be supplied to the patient, is connected to upstream branch tube (14). Then, with the indwelling needle inserted and remaining in the patient, the medicine is administered to the patient by operating the operating part (20) to send the medicine from the container or the like to the patient. To administer another medicine or the like to the patient in addition to medicine supplied from a container or the like, the other medicine is injected into chamber part (11) from flow merging branch tube (12) through a transfusion tube connected to adaptor (27).

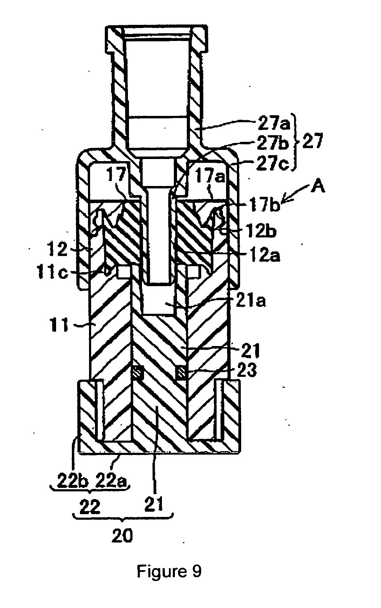

That is, by operating the operating part (20) so that guiding projection (19) is positioned at the end of horizontal guide (25c) with adaptor (27) attached to flow merging branch tube (12), as shown in Figures 8 and 9, female luer part (27a) of adaptor (27) and downstream branch tube (13) are connected through chamber part (11). In this case, upstream branch tube (14) is disconnected from the inside of chamber part (11) because the top end of valve body (21) is tight against rubber stopper (17). Mark (24a) that indicates that flow merging branch tube (12) and downstream branch tube (13) are connected appears in window (18) of chamber part (11), as shown in Figure 1(b). In addition, projection (19) is secured at the end of horizontal guide (25c) by narrow part (26b).

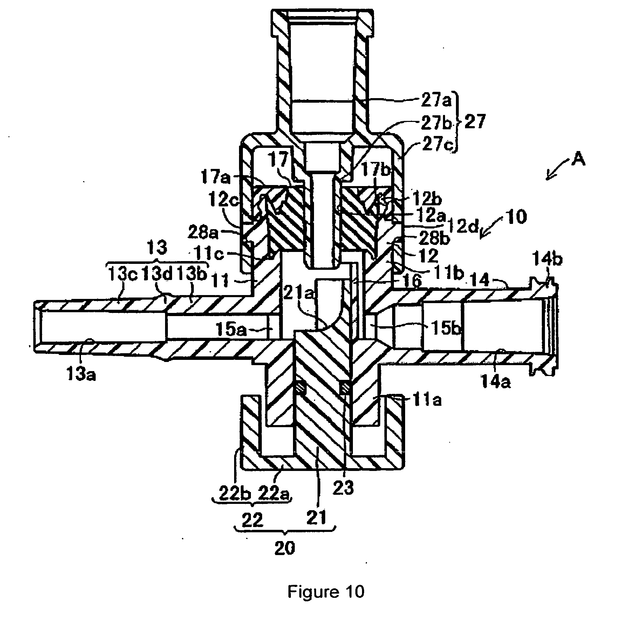

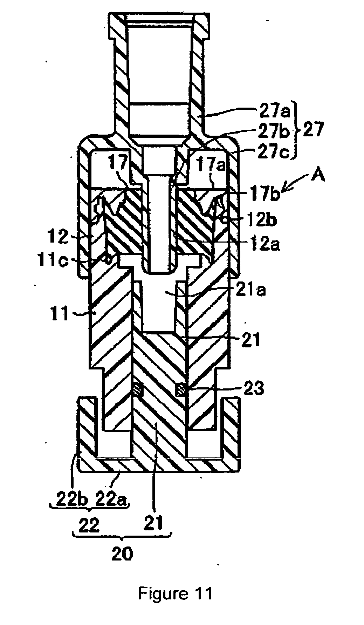

When operating part (20) is operated so that guiding projection (19) is positioned at the end of horizontal guide (25b), as shown in Figures 10 and 11, female luer part (27a) of adaptor (27), downstream branch tube (13) and upstream branch tube (14) are all connected through chamber part (11). At this time, the top end of valve body (21) is separated from rubber stopper (17). To indicate that flow merging branch tube (12) and downstream branch tube (13), and upstream branch tube (14) and downstream branch tube (13) are connected, mark (24b) appears in window (18) of chamber part (11), as shown in Figure 2(b). In addition, guiding projection (19) is secured at the end of horizontal guide (25b) by narrow part (26a).

Here, operation of operating part (20) in this case is accomplished from the state in Figure 1(b), by turning operating part (20) clockwise, as viewed from the bottom surface, so that guiding projection (19) is positioned at the bottom end of vertical guide (25a), and also moving operating part (20) downward so that guiding projection (19) is positioned at the top end of vertical guide (25a). Then operating part (20) is turned counterclockwise, as viewed from the bottom surface, so that guiding projection (19) is positioned at the end of horizontal guide (25b), yielding the state in Figure 2(b). The reverse operation is performed to go from the state in Figure 2(b) to the state in Figure 1(b).

In this way, with liquid co-infusion device (A) pertaining to this embodiment, an operating part (20) is provided that can move up and down in the axial direction of chamber part (11). By manipulating operating part (20) up and down, downstream branch tube (13) and upstream branch tube (14) can be connected and disconnected by valve body (21). It is also constituted so that an adaptor (27) can be detachably attached to flow merging branch tube (12) formed at the top of chamber part (11), and adaptor (27) can be connected to the inside of chamber part (11) by attaching adaptor (27).

Therefore, one or two types of medicine or the like can be selected and administered as appropriate to the patient. With liquid co-infusion device (A), the operation to switch between connection and disconnection of downstream branch tube (13) and upstream branch tube (14) is accomplished by moving operating part (20) up and down, so it is simple and mishandling is also unlikely to occur. In this case, operating part (20) only advances or retreats relative to chamber part (11), so that operating part (20) does not project from the circumferential surface of chamber part (11) to get in the way. In addition, when liquid co-infusion device (A) is placed on a bed or the like, the axial direction of chamber (11) is horizontal, so that operating part (20) will not contact the patient causing the operating position to be switched.

Also, with liquid co-infusion device (A) pertaining to the present invention, a guide slot (25) is furnished in grip part (22) of operating part (20), and a guiding projection (19) is also furnished in small-diameter part (11a) of chamber part (11). Operating part (20) moves relative to chamber part (11) by moving guide slot (25) relative to guiding projection (19), allowing switching between connection and disconnection of downstream branch tube (13) and upstream branch tube (14). For this reason, operating part (20) moves along a precise path and channel switching is reliable.

Operating part (20) also has a grip part (22) that can move along the outer circumferential surface of chamber part (11), so that operation of operating part (20) is easy. In addition, narrow parts (26a) and (26b) are furnished near the two ends of guide slot (25), so that guiding projection (19) is securely locked by narrow parts (26a) and (26b), so that it will not move. Because of this, guiding projection (19) is prevented from moving unintentionally and the channel from being changed. A window (18) is also formed in chamber part (11), and marks (24a) and (24b) are furnished on the section of the circumferential surface of valve body (21) exposed in window (18) to indicate the connected channels when valve body (21) is positioned toward the inside in the axial direction of chamber part (11), and on the section exposed in window (18) when valve body (21) is positioned toward the outside in the axial direction of chamber part (11), respectively. For this reason, the operator will not confuse the channels that are connected or disconnected.

### Second embodiment

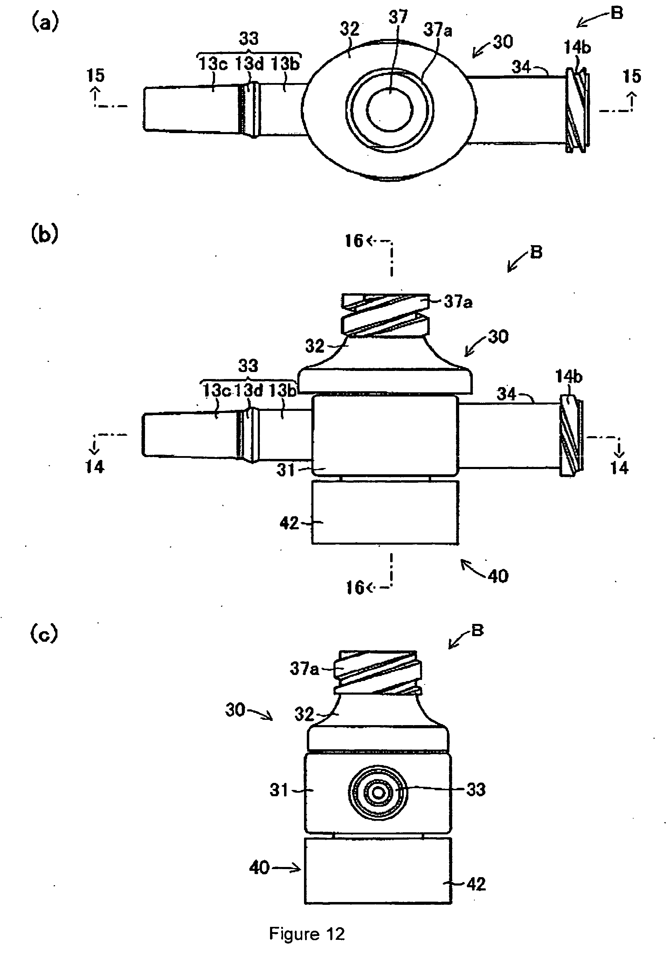

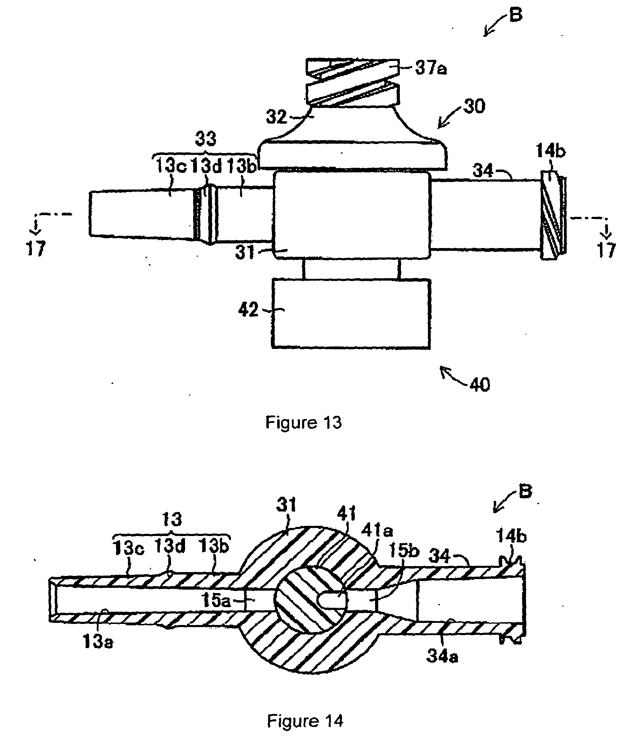

Figures 12 and 13 show a liquid co-infusion device (B) pertaining to a second embodiment of the present invention. The liquid co-infusion device (B) is constituted with a liquid co-infusion device body (30) and an operating part (40), and the inside is constituted as shown in Figures 14-18. Specifically, flow merging branch tube (32) formed at the top of chamber part (31) is constituted with a member that is separate from chamber part (31) and is constituted with a tapered cylinder body such that the diameter toward chamber part (31) is larger and the diameter becomes smaller going upward. The shape of flow merging branch tube (32), in plan view, is elliptical.

A lockable part (32a) is formed at the bottom end on the inner circumferential surface of merging branch tube (32), and merging branch tube (32) is connected to chamber part (31) by locking lockable part (32a) onto locking part (31 a) formed on the outer circumferential surface at the top end of chamber part (31). A rubber stopper (37) is inserted into the opening at the top end of merging branch tube (32), and rubber stopper (37) is secured from coming loose from the opening in merging branch tube (32) by cover (37a). A channel (37b) is formed in rubber stopper (37) that extends upward from the outer circumferential section of the lower surface, and rubber stopper (37) is locked to the top end of merging branch tube (32) by inserting the top end of merging branch tube (32) into groove (37b).

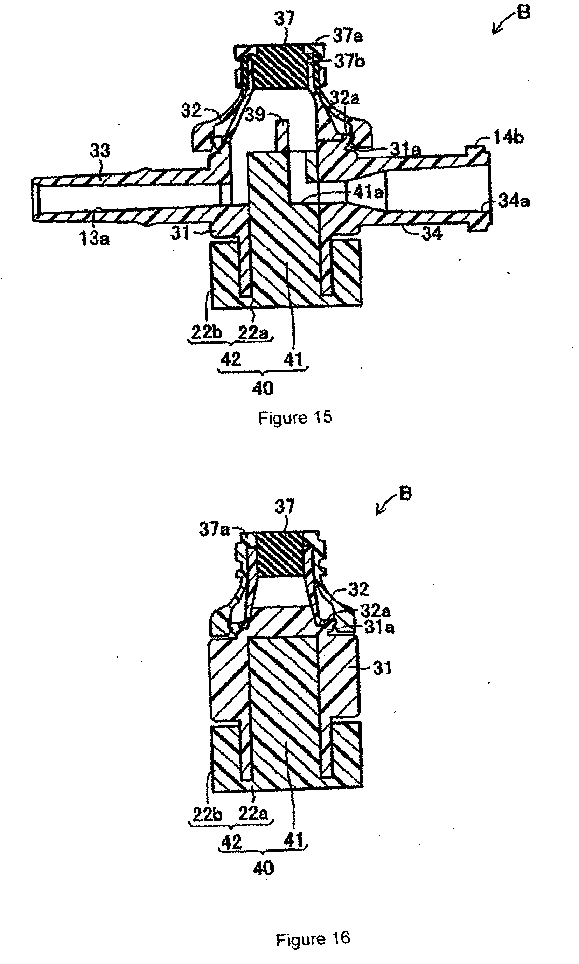

Rubber stopper (37) is secured to the top of flow merging branch tube (32) by tightening the outer circumferential part of the top surface and the top part of the circumferential surface using cover (37a). Here, a slit (not shown) for forming a channel in flow merging branch tube (32) is also furnished in rubber stopper (37). A wall (39) is formed inside chamber part (31) in a section toward the top. Medicine or the like flowing inside chamber part (31) passes through the top of the interior of chamber part (31) when in the state shown in Figure 15 because of wall (39). Because of this, air or the like can be prevented from remaining in chamber part (31).

Operating part (40) is constituted with a valve body (41) that is approximately a round column, and a grip part (42) that is connected to the bottom end of valve body (41). A channel (41 a) in the section of valve body (41) facing upstream branch tube (34) turns at a right angle, after extending from upstream branch tube (34), toward downstream branch tube (33) to extend upward toward flow merging branch tube (32). The bottom end of channel (41a), as shown in Figure 15, is at a position wherein it connects to channel (34a) in upstream branch tube (34) when valve body (41) is in the up position, and upstream branch tube (34) is connected to the inside of chamber part (31). In this case, the top end surface of valve body (41) touches wall (39).

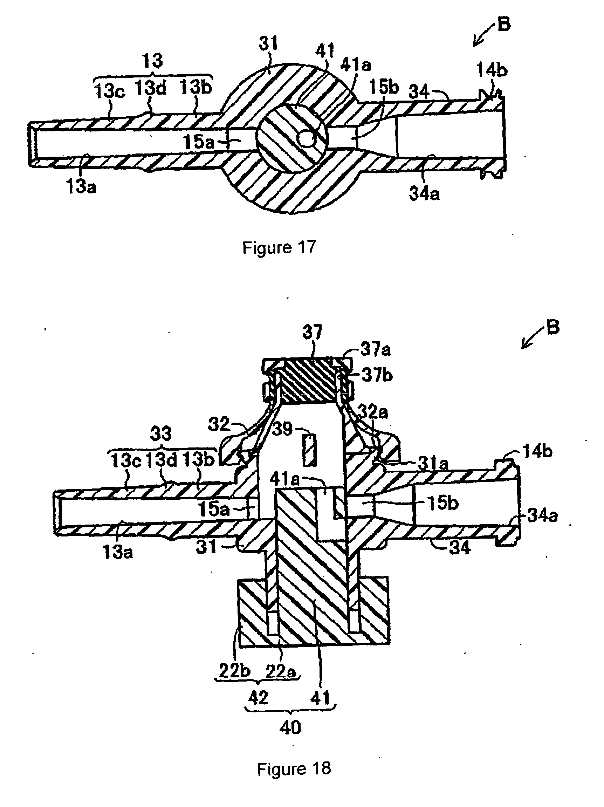

As shown in Figure 18, the bottom end of channel (41 a) is below channel (34a) of upstream branch channel (34) when valve body (41) is in the down position, and the channel is blocked by the inner circumferential surface of chamber part (31). In this case, the top end surface of valve body (41) is separated from and below wall (39). For this reason, as shown in Figure 15, when valve body (41) is in the up position, downstream branch tube (33) and upstream branch tube (34) are connected through chamber part (31). Also, as shown in Figure 18, when valve body (41) is in the down position, downstream branch tube (33) and upstream branch tube (34) are disconnected by valve body (41).

An adaptor that is the same as adaptor (27) in the abovementioned first embodiment can also be detachably attached to flow merging branch tube (32). Here, no window, marks, guide slots or guiding projections are furnished for liquid co-infusion device (B). Operating part (40) in liquid co-infusion device (B) is placed in a specific position using the frictional resistance between it and chamber part (31). The constitution of the other sections of liquid co-infusion device (B) is the same as for said liquid co-infusion device (A). Therefore, the same symbols denote the same sections, and explanations are omitted.

In this constitution, when a specific medicine is administered to a patient, a transfusion tube connected to an indwelling needle is connected to downstream branch tube (33) and a transfusion tube, extending from a container or the like in which a medicine is stored, is connected to upstream branch tube (34). Then, with the indwelling needle inserted and remaining in the patient, operating part (40) is operated and medicine is administered to the patient by sending the medicine from the container or the like toward the patient. When another medicine or the like is administered to the patient in addition to the medicine supplied form the container or the like, the other medicine is injected into chamber (31) from flow merging branch tube (32) through a transfusion tube connected to the adaptor.

That is, when operating part (40) is in the up position, with the adaptor detached from flow merging branch tube (32), as shown in Figures 14-16, channel (41 a) in valve body (41) is connected to channel (34a) in upstream branch tube (34), and upstream branch tube (34) is connected to chamber part (31). When operating part (40) is in the down position, as shown in Figures 17 and 18, channel (41 a) in valve body (41) is blocked by the inner circumferential surface of chamber part (31), and upstream branch tube (34) and chamber part (31) are disconnected.

In addition, when the adaptor is attached to converting branch tube (32), the adaptor and downstream branch tube (33) can be connected through chamber part (31). In this case, when channel (41 a) in valve body (41) and channel (34a) in upstream branch tube (34) are left connected, medicine or the like can be supplied to downstream branch tube (33) from both the adaptor and upstream branch tube (34). When channel (41 a) in valve body (41) and channel (34a) in upstream branch tube (34) are left disconnected, liquid medicine or the like can be supplied to downstream branch tube (33) only from the adaptor. Since liquid co-infusion device (B) is constituted as described above, the structure is simple and operation is also simple. Otherwise, the functioning and effects of liquid co-infusion device (B) are the same as in the abovementioned liquid co-infusion device (A).

The liquid co-infusion device pertaining to the present invention is not limited to the abovementioned embodiments, and modifications can be implemented as appropriate. For example, with the abovementioned first embodiment, guide slot (25) is constituted with a vertical guide (25a) that extends vertically and horizontal guides (25b) and (25c) that extend in parallel horizontally, and narrow parts (26a) and (26b) are formed at the ends of horizontal guides (25b) and (25c), respectively, but the guide slot could also be constituted with only a vertical guide (25a). In this case, a narrow part is formed near each end of vertical guide (25a). The guide groove can also be constituted with a slot that is furnished only in the inner circumferential surface of grip part (22) rather than with a long hole through the inner and outer surfaces of grip part (22), so as not to be visible from the outside.

In addition, the guide slot and guiding projections are in facing sections of the chamber part and the operating part, but they can also be furnished in other sections. The guiding part and guided part can also be constituted with other than a guide slot and a guiding projection. In addition, a transparent member can also be furnished for window (18) so that the inside is visible. With the abovementioned application examples, a slit (12a) or the like is furnished in rubber stopper (17) or (37) and insertion part (27b) is inserted into slit (12a), with an adaptor (27) or the like being attached to flow merging branch tube (12) or (32), but the male luer part of a syringe or an injection needle can also be inserted into rubber stopper (17) or (37) in place of adaptor (27). Here, when an injection needle is inserted, it is not necessary to provide slit (12a) in rubber stopper (17) or (37). In addition, the shape, materials, and the like of other sections that constitute the liquid co-infusion device can also be modified and implemented as appropriate.

## Claims

1. A liquid co-infusion device (A, B) having a body (10) comprising a chamber (11), having a first connection port (12) formed at one end in an axial direction of said chamber, and second and third connection ports (13, 14) extending sidewards from said chamber,
and an operating part (20), arranged at a second end in the axial direction of said chamber (11) and movable in the axial direction of said chamber, wherein movement of the operating part in said axial direction switches between connection and disconnection of one of said connection ports (14) to an interior of said chamber wherein when said operating part is in a first end position, said first connection port (12) is in fluid flow connection to said second connection port (13),
**characterized in that** when said operating part is in a second end position said first connection port (12) and said third connection port (14) are in fluid flow connection with said second connection port (13).

2. The liquid co-infusion device according to Claim 1, wherein said operating part includes an outer sidewall (22b) arranged to be slidably movable over a outer surface portion (11a) of said chamber and wherein said outer sidewall and said outer surface portion include mutually co-operating guide parts (19, 25) arranged so as to guide the movement of said operating part.

3. The liquid co-infusion device according to Claim 2, wherein the mutually co-operating guide parts comprise a guide slot (25) and a projection (19) arranged to move in said guide slot.

4. The liquid co-infusion device according to Claim 3 wherein said guide slot (25) is arranged to include an axially extending part (25a) and at least one part (25b, c) extending laterally to said axially extending part so as to selectively allow axial movement of said operating part and to prevent axial movement of said operating part respectively.

5. The liquid co-infusion device according to Claim 1 wherein a window (18) is provided in a wall of said chamber and wherein said operating part (20) includes markings (24a, b) thereon such that an operating state of said co-infusion device is displayed in said window.

6. The liquid co-infusion device according to Claim 1 wherein said operating part includes a through channel (21 a) therein, wherein movement of said operating part in said axial direction displaces said through channel between a fluid flow position relative to said third connection port (14) and a fluid sealing position relative to said third connection port.

7. The liquid co-infusion device according to Claim 1, wherein said operating part includes a groove (21a) at an end thereof disposed within said chamber and wherein said groove is bounded by a sidewall such that when said operating part is moved in said axial direction into said chamber, said sidewall provides a fluid flow seal to cause said disconnection.

8. The liquid co-infusion device according to Claim 7, wherein said first connection port incorporates an elastic sealing element (17) and said operating part sidewall is moveable to provide a fluid flow blocking seal between said sidewall and said sealing element.

9. The liquid co-infusion device according to Claim 8, wherein said groove is shaped to accommodate an end of a connector (27) inserted through said elastic sealing element when said operating part is positioned to as to cause said disconnection.

## Patentansprüche

1. Flüssigkeits-Koinfusionsgerät (A, B) mit einem Körper (10), der eine Kammer (11) umfasst, die einen ersten Verbindungsstutzen (12), der an einem Ende in einer Axialrichtung der Kammer ausgebildet ist, und einen zweiten und einen dritten Verbindungsstutzen (13, 14), die sich von der Kammer seitwärts erstrecken, aufweist,
und einem Betriebsteil (20), der an einem zweiten Ende in Axialrichtung der Kammer (11) angeordnet und in der Axialrichtung der Kammer beweglich ist, wobei eine Bewegung des Betriebsteils in der Axialrichtung zwischen Verbindung und Trennung eines der Verbindungsstutzen (14) mit einem Inneren der Kammer schaltet, wobei der erste Verbindungsstutzen (12) mit dem zweiten Verbindungsstutzen (13) in Strömungsverbindung steht, wenn sich der Betriebsteil in einer ersten Endposition befindet,
**dadurch gekennzeichnet, dass** der erste Verbindungsstutzen (12) und der dritte Verbindungsstutzen (14) in Strömungsverbindung mit dem zweiten Verbindungsstutzen (13) stehen, wenn sich der Betriebsteil in einer zweiten Endposition befindet.

2. Flüssigkeits-Koinfusionsgerät nach Anspruch 1, wobei der Betriebsteil eine äußere Seitenwand (22b) enthält, die so angeordnet ist, dass sie über einen Außenflächenteil (11a) einer Kammer verschiebbar ist, und wobei die äußere Seitenwand und der Außenflächenteil zusammenwirkende Führungsteile (19, 25) enthalten, die so angeordnet sind, dass sie die Bewegung des Betriebsteils führen.

3. Flüssigkeits-Koinfusionsgerät nach Anspruch 2, wobei die zusammenwirkenden Führungsteile einen Führungsschlitz (25) und einen Vorsprung (19) umfassen, die so angeordnet sind, dass sie sich in dem Führungsschlitz bewegen.

4. Flüssigkeits-Koinfusionsgerät nach Anspruch 3, wobei der Führungsschlitz (25) so angeordnet ist, dass er einen sich axial erstreckenden Teil (25a) und mindestens einen Teil (25b, c), der sich lateral zu dem sich axial erstreckenden Teil erstreckt, enthält, um gezielt eine axiale Bewegung des Betriebsteils zu gestatten bzw. eine axiale Bewegung des Betriebsteils zu verhindern.

5. Flüssigkeits-Koinfusionsgerät nach Anspruch 1, wobei ein Fenster (18) in einer Wand der Kammer vorgesehen ist und wobei der Betriebsteil (20) Markierungen (24a, b) daran enthält, so dass ein Betriebszustand des Koinfusionsgeräts in dem Fenster angezeigt wird.

6. Flüssigkeits-Koinfusionsgerät nach Anspruch 1, wobei der Betriebsteil einen Durchgangskanal (21 a) darin enthält, wobei eine Bewegung des Betriebsteils in der Axialrichtung den Durchgangskanal zwischen einer Fluidstromposition bezüglich des dritten Verbindungsstutzens (14) und einer Fluidabdichtposition bezüglich des dritten Verbindungsstutzens verschiebt.

7. Flüssigkeits-Koinfusionsgerät nach Anspruch 1, wobei der Betriebsteil an einem Ende davon eine Nut (21 a) enthält, die in der Kammer angeordnet ist, und wobei die Nut durch eine Seitenwand so begrenzt wird, dass die Seitenwand eine Fluidstromdichtung bereitstellt, um eine Trennung zu verursachen, wenn der Betriebsteil in der Axialrichtung in die Kammer bewegt wird.

8. Flüssigkeits-Koinfusionsgerät nach Anspruch 7, wobei der erste Verbindungsstutzen ein elastisches Dichtungselement (17) enthält und die Betriebsteilseitenwand beweglich ist, um eine Fluidstromsperrdichtung zwischen der Seitenwand und dem Dichtungselement bereitzustellen.

9. Flüssigkeits-Koinfusionsgerät nach Anspruch 8, wobei die Nut zur Aufnahme eines Endes eines Verbinders (27) geformt ist, der durch das elastische Dichtungselement geschoben wird, wenn der Betriebsteil so positioniert ist, dass er die Trennung verursacht.

## Revendications

1. Dispositif de co-infusion liquide (A, B) ayant un corps (10) comprenant une chambre (11), ayant un premier orifice de connexion (12) formé à une extrémité dans une direction axiale de ladite chambre et des deuxième et troisième orifices de connexion (13, 14) s'étendant latéralement depuis ladite chambre,
et une partie fonctionnelle (20), agencée à une deuxième extrémité dans la direction axiale de ladite chambre (11) et pouvant être déplacée dans la direction axiale de ladite chambre, le mouvement de la partie fonctionnelle dans ladite direction axiale commutant entre la connexion et la déconnexion de l'un desdits orifices de connexion (14) à un intérieur de ladite chambre, ledit premier orifice de connexion (12) étant en communication d'écoulement fluidique avec ledit deuxième orifice de connexion (13) lorsque ladite partie fonctionnelle est dans une première position d'extrémité,
**caractérisé en ce que** lorsque ladite partie fonctionnelle est dans une deuxième position d'extrémité, ledit premier orifice de connexion (12) et ledit troisième orifice de connexion (14) sont en communication d'écoulement fluidique avec ledit deuxième orifice de connexion (13).

2. Dispositif de co-infusion liquide selon la revendication 1, dans lequel ladite partie fonctionnelle comporte une paroi latérale extérieure (22b) agencée de manière à pouvoir être déplacée par coulissement par-dessus une portion de surface extérieure (11a) de ladite chambre et ladite paroi latérale extérieure et ladite portion de surface extérieure comportent des parties de guidage coopérant mutuellement (19, 25) agencée de manière à guider le mouvement de ladite partie fonctionnelle.

3. Dispositif de co-infusion liquide selon la revendication 2, dans lequel les parties de guidage coopérant mutuellement comprennent une fente de guidage (25) et une saillie (19) agencée de manière à se déplacer dans ladite fente de guidage.

4. Dispositif de co-infusion liquide selon la revendication 3, dans lequel ladite fente de guidage (25) est agencée de manière à comporter une partie s'étendant axialement (25a) et au moins une partie (25b, c) s'étendant latéralement à ladite partie s'étendant axialement de manière à permettre un mouvement axial sélectif de ladite partie fonctionnelle et à empêcher un mouvement axial de ladite partie fonctionnelle, respectivement.

5. Dispositif de co-infusion liquide selon la revendication 1, dans lequel une fenêtre (18) est prévue dans une paroi de ladite chambre et dans lequel ladite partie fonctionnelle (20) comporte des marquages (24a, b) sur elle, de telle sorte qu'un état fonctionnel dudit dispositif de co-infusion soit affiché dans ladite fenêtre.

6. Dispositif de co-infusion liquide selon la revendication 1, dans lequel ladite partie fonctionnelle comporte un canal traversant (21 a), le mouvement de ladite partie fonctionnelle dans ladite direction axiale déplaçant ledit canal traversant entre une position d'écoulement fluidique par rapport audit troisième orifice de connexion (14) et une position de blocage fluidique par rapport audit troisième orifice de connexion.

7. Dispositif de co-infusion liquide selon la revendication 1, dans lequel ladite partie fonctionnelle comporte une rainure (21 a) à l'une de ses extrémités, disposée dans ladite chambre et ladite rainure est bordée par une paroi latérale de telle sorte que lorsque ladite partie fonctionnelle est déplacée dans ladite direction axiale dans ladite chambre, ladite paroi latérale fournisse un blocage de l'écoulement fluidique de manière à provoquer ladite déconnexion.

8. Dispositif de co-infusion liquide selon la revendication 7, dans lequel ledit premier orifice de connexion comprend un élément de blocage élastique (17) et la paroi latérale de ladite partie fonctionnelle est déplaçable de manière à fournir un joint de blocage de l'écoulement fluidique entre ladite paroi latérale et ledit élément de blocage.

9. Dispositif de co-infusion liquide selon la revendication 8, dans lequel ladite rainure est formée de manière à recevoir une extrémité d'un connecteur (27) inséré à travers ledit élément de blocage élastique lorsque ladite partie fonctionnelle est positionnée de manière à provoquer ladite déconnexion.
